(19) Europäisches Patentamt
European Patent Office
Office européen des brevets

(11) Numéro de publication : **0 380 410 B1**

(12) **FASCICULE DE BREVET EUROPEEN**

(45) Date de publication du fascicule du brevet : **07.04.93 Bulletin 93/14**

(51) Int. Cl.[5] : **C07C 315/02,** C07C 317/18, B01F 17/00, C07D 407/12

(21) Numéro de dépôt : **90400197.1**

(22) Date de dépôt : **24.01.90**

# (54) Procédé de préparation de tensio-actifs non-ioniques à partir de l'isopropylidène-1,2 époxypropyl-3 glycérol et d'un mercaptan, nouveaux produits tensio-actifs et leur utilisation.

(30) Priorité : **26.01.89 FR 8900962**

(43) Date de publication de la demande : **01.08.90 Bulletin 90/31**

(45) Mention de la délivrance du brevet : **07.04.93 Bulletin 93/14**

(84) Etats contractants désignés : **AT BE CH DE DK ES FR GB IT LI NL SE**

(56) Documents cités :
**EP-A- 0 042 505**
**EP-A- 0 066 107**
**DE-A- 1 961 731**
**FR-A- 1 484 723**

(56) Documents cités :
**FR-A- 1 561 585**
**FR-A- 2 328 764**
**FR-A- 2 482 128**
**US-A- 3 880 766**
**US-A- 4 058 629**

(73) Titulaire : **L'OREAL**
**14, Rue Royale**
**F-75008 Paris (FR)**

(72) Inventeur : **Sebag, Henri**
**26, rue Erlanger**
**F-75016 Paris (FR)**

(74) Mandataire : **Casalonga, Axel et al**
**BUREAU D.A. CASALONGA - JOSSE**
**Morassistrasse 8**
**W-8000 München 5 (DE)**

**EP 0 380 410 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention a pour objet un nouveau procédé de préparation de composés non ioniques, de nouveaux produits tensio-actifs, les compositions les contenant ainsi que l'utilisation de ces compositions dans l'industrie des cosmétiques, des produits pharmaceutiques et des produits textiles.

Le nouveau procédé consiste dans une première étape, à condenser sur des composés comportant un groupement thiol, l'isopropylidène-1,2 époxydropyl-3 glycérol de formule (II) :

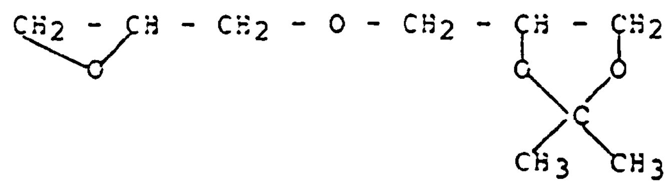

(II)

puis dans une deuxième étape, à hydrolyser les produits obtenus, pour préparer des produits non-ioniques polyhydroxylés tensio-actifs.

On connaît des composés tensio-actifs non-ioniques polyhydroxylés. En particulier, le brevet français n°1 484 723 et le brevet US n° 3 674 902 décrivent des composés tensio-actifs de formule :

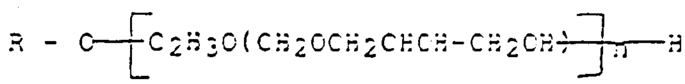

($F_1$)

Ces produits de formule ($F_1$) sont obtenus par réaction de l'allylglycidyléther avec un alcool gras saturé ou insaturé, puis, par hydroxylation des doubles liaisons avec de l'eau oxygénée à 130 volumes, en présence d'acide formique à 98% pendant 24 à 48 heures à 40°C. L'utilisation d'eau oxygénée et d'acide concentrés présente toujours des risques tant du point de vue de la sécurité, que du point de vue de la formation des produits dus à une peroxydation ou dérivés de la réaction secondaire d'époxydation. Il n'est pas possible, selon ce procédé, d'utiliser comme matière de départ, à la place des alcools, des mercaptans, car lors de l'étape d'hydroxylation avec de l'eau oxygénée, celle-ci oxyderait les fonctions thio-éther en fonctions sulfoxyde.

EP-A-0066107 enseigne la préparation d'un monoéther spécifique du diglycérol pouvant être préparé par réaction d'un alcool et d'un époxyde du type 1,3-dioxolane, en présence d'un catalyseur, le produit d'addition étant soumis à une hydrolyse.

Cette demande de brevet n'enseigne pas la préparation de thioéthers.

DE-A-1.961.731 enseigne la préparation de polyhydroxyéthers par réaction d'un composé ayant un atome d'hydrogène réactif, tel qu'un alcool, phénol ou mercaptan avec le tertiobutylglycidyléther. Le procédé selon ce document utilise comme matière de départ surtout des alcools. Lorsque la matière de départ est un mercaptan, il n'enseigne que la préparation de produits de mono-addition.

FR-A-1.561.585 a pour objet les produits d'addition d'oxyde d'éthylène et/ou de propylène sur des composés organiques contenant du soufre.

FR-A-2.328.764 enseigne un procédé de préparation d'agents de surface non-ioniques à partir de la monochlorhydrine du glycérol et d'un mercaptan.

FR-A-4.058.629 a pour objet la préparation d'un monosulfoxyde polyhydroxylé par condensation du glycidol sur un mercaptan.

Les références ci-dessus enseignent seulement un procédé de mono-addition d'un composé époxyde sur un alcool ou un mercaptan. La présente invention a pour but un procédé de poly-addition d'un composé époxyde spécifique, à savoir l'isopropylidène-1,2 époxypropyl-3 glycérol (IEPG) sur un mercaptan.

Le procédé de la présente invention consiste à préparer par réaction de l'isopropylidène-1,2 époxypropyl-3 glycérol de formule (II) avec un mercaptan de formule ($I_1$), des tensio-actifs non-ioniques polyhydroxylés de formule ($IV_1$) selon le schéma réactionnel suivant :

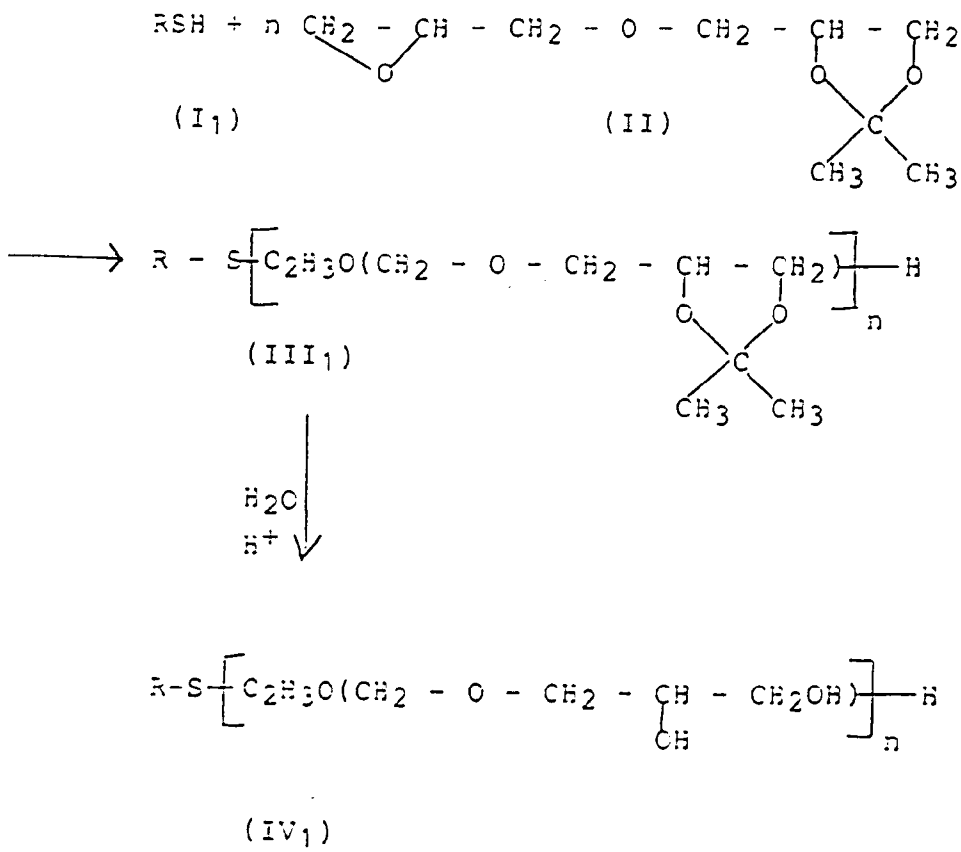


(I1) (II) (III1) (IV1)

Le groupement

$$[C_2H_3\ O\ (CH_2 - O - CH_2 - CHOCH - CH_2OH)]$$

désigne l'un ou l'autre des groupements suivants qui résultent des deux sens d'ouverture possibles de la fonction époxyde de (II).

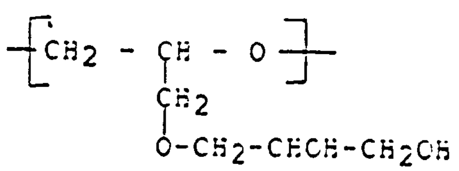 et 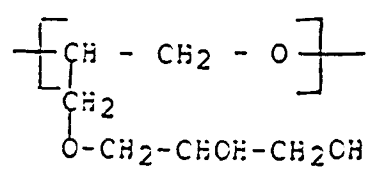

Les composés ($IV_1$) peuvent ensuite éventuellement être oxydés en sulfoxydes ($V_1$) selon un procédé connu, avec de l'eau oxygénée.

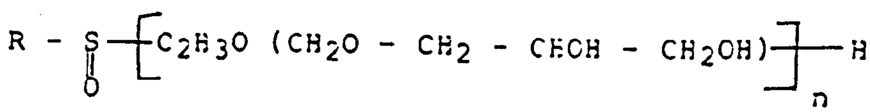

($V_1$)

Le procédé selon l'invention permet de préparer des tensio-actifs non-ioniques comportant des chaînes grasses à nombre élevé d'atomes de carbone, de $C_8$ à $C_{36}$.

Les produits ainsi préparés sont des mélanges statistiques de composés homologues constitués d'une chaîne grasse et d'un enchaînement de motifs hydrophiles en nombre inférieur, égal ou supérieur au nombre

n de moles d'époxyde de formule (II) mises en oeuvre par mole de ($I_1$).

L'avantage du procédé de l'invention est qu'il peut être utilisé avec des composés à hydrogène actif de haut poids moléculaire et que dans le mélange de produits obtenus, il ne reste plus de réactifs ($I_1$), c'est-à-dire que l'homologue le moins condensé comporte au minimum un enchaînement de deux motifs glycérol.

Le composé de formule ($I_1$) peut comporter en plus du groupement thiol, entre autres, un groupement hydroxyle en position 2.

Ce dernier est également susceptible de réagir avec l'époxyde de formule (II) initiant ainsi une seconde chaîne hydrophile.

Les composés de départ de formule globale ($I_1$) peuvent être présentés sous une formule générale (I) plus précise, à savoir :

$$R_1 - (X)_t \left[ CH_2 - CH_2\,O \right]_m CH_2 - \left( \underset{OH}{\underset{|}{CH}} \right)_u - \left( \underset{R_2}{\underset{|}{CH}} \right)_v - SH \qquad (I)$$

dans laquelle :

- $R_1$ désigne un radical hydrocarboné aliphatique, cycloaliphatique ou alkylarylique en $C_8$ à $C_{36}$,
- $R_2$ désigne un radical alcoyle ou alcoxyméthyle en $C_8$ à $C_{21}$ ou un atome d'hydrogène,
- X désigne -O- ou -S- ,
- m désigne zéro ou un nombre entier ou décimal de 1 à 20,
- t, u et v désignent zéro ou 1.

Quand u = 1, v est obligatoirement égal à 1 et $R_2$ = H, et

quand v = 1 et $R_2$ ne désigne pas H, u est égal à zéro.

La somme des atomes de carbone $R_1$ et $R_2$ est au moins de 8 et au plus de 36.

La formule générale (V) regroupant les produits de l'invention peut ainsi s'écrire

$$R_1(X_1)_t \left[ CH_2 - CH_2\,O \right]_m CH_2 - \left( \underset{OB}{\underset{|}{CH}} \right)_u - \left( \underset{R_2}{\underset{|}{CH}} \right)_v - \underset{(O)_w}{\underset{\|}{S}} - A \qquad (V)$$

dans laquelle :

- $R_1$, $R_2$, m, t, u, v, ont la même signification que pour la formule (I) ; w=O ou 1,
- $X_1$ a la même signification que X et peut de plus désigner S=O quand w=1,
- A désigne l'enchaînement

$$\left[ C_2H_3\,O\,(CH_2 - O - CH_2 - CHOH - CH_2OH) \right]_a H$$

et B désigne l'enchaînement

$$\left[ C_2H_3\,O\,(CH_2 - O - CH_2 - CHOH - CH_2OH) \right]_b H$$

a désigne un nombre entier ou décimal de 1,5 à 8

b désigne zéro ou un nombre entier ou décimal tel que a+b = n

n est un nombre entier ou décimal de 1,5 à 8, et représente le nombre de moles de composés de formule (II) mises en oeuvre par mole de composé à hydrogène actif de formule (I).

La réaction de polyaddition de l'époxyde (II) au réactif (I) est réalisée en présence d'un catalyseur basique comme le méthylate ou l'éthylate de sodium ou de potassium ou le t-butylate de potassium, de préférence sous atmosphère inerte comme l'azote.

Les quantités de catalyseur sont de 3 à 10% molaires par rapport au réactif de formule (I).

Le groupement thiol (mercaptan) est nettement plus réactif vis-à-vis de l'époxyde de formule (II) que le groupement hydroxyle éventuel ou que celui qui se forme après ouverture du groupe oxirane. Le groupement thiol peut réagir entre 40 et 90°C.

La première mole de composé (II) par mole de composé (I) et qui se fixe sur le groupement thiol est donc ajoutée à une température comprise entre 40 et 90°C et conduit aux produits de mono-addition de formule (V) dans laquelle a=1 et b=0.

Cette réaction peut être faite en présence d'un solvant polaire, par exemple un alcool en $C_1$ à $C_4$, comme le méthanol, l'éthanol, l'isopropanol ou le t-butanol ou d'un solvant non polaire comme le toluène.

Le reste de l'époxyde de formule (II) qui réagit avec le(s) groupement(s) alcool est introduit progressivement entre 130 et 160°C après avoir éliminé le solvant éventuel.

L'époxyde est introduit en 1 à 3 heures et le chauffage est maintenu encore pendant 1 à 3 heures jusqu'à consommation complète de l'époxyde de formule (II).

Le mélange statistique de composés intermédiaires de formule générale (III) est ensuite hydrolysé, de préférence en solution hydro-alcoolique, en présence d'un acide minéral comme l'acide chlorhydrique, sulfurique ou phosphorique ou en présence d'un acide organique comme l'acide acétique, l'acide lactique ou l'acide méthane sulfonique. L'alcool utilisé comme cosolvant pour la réaction d'hydrolyse, peut être du méthanol, de l'éthanol ou de l'isopropanol.

Après la réaction d'hydrolyse, la masse réactionnelle est neutralisée puis elle est chauffée sous pression réduite pour éliminer les produits volatils.

Dans le cas ou w=1, les produits ainsi obtenus sont oxydés à l'eau oxygénée, selon un procédé connu, à une température comprise entre 20° et 50°C.

Les mercaptans de départ de formule (I) sont choisis parmi les alcoyl mercaptans, les alcoyloxypolyéthoxyéthyl mercaptans, les alcoylphénoxypolyéthoxyéthyl mercaptans, les alcoylthiopolyéthoxyéthyl mercaptans, les 2-hydroxyalcoyl mercaptans, les 3-alcoyloxy 2-hydroxypropyl mercaptans, les 1-alcoyloxy méthylalcoyl mercaptans, les 1,3-dialcoyloxypropyl-2 mercaptans.

Ces mercaptans peuvent être préparés par des procédés connus par condensation de l'acide thio-acétique ou de son sel de sodium ou de potassium avec :

- les dérivés halogénés de formule $R_1X$, où $R_1$ a les significations ci-dessus indiquées;
- un ester méthane sulfonique $R_3$-O-$SO_2CH_3$, où $R_3$ désigne un radical aliphatique ou cycloaliphatique en $C_8$-$C_{36}$;
- un ester para-toluène sulfonique de formule :

$$R_3 - O - SO_2 - C_6H_5 - CH_3$$

  où $R_3$ désigne un radical aliphatique ou cycloaliphatique en $C_8$-$C_{36}$;
- un ester méthane sulfonique ou para-toluène sulfonique d'alcool polyéthoxylé ou d'alcoylphénol polyéthoxylé, de formules :

$$R_1(OCH_2CH_2)_m-OSO_2-CH_3, \text{ ou}$$
$$R_1(OCH_2CH_2)_m-O-SO_2-C_6H_5-CH_3$$

  où $R_1$ a les significations ci-dessus indiquées;
- un ester méthanesulfonique d'un alcoyl thio éthanol polyéthoxylé de formule :

$$R_3-S-CH_2-CH_2(OCH_2CH_2)_m-OSO_2-CH_3$$

  où $R_3$ désigne un radical aliphatique en $C_8$-$C_{36}$,

  toutes ces condensations étant suivies d'hydrolyse.

Les mercaptans de formule (I) peuvent également être préparés par réaction d'addition de l'acide thioacétique sur un dérivé insaturé de formule :

$$R_5 - CH = CH_2$$

où $R_5$ désigne un radical aliphatique en $C_6$ à $C_{34}$
ou sur un époxyde de formule :

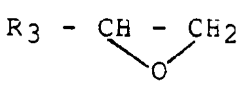

où $R_3$ désigne un radical aliphatique en $C_8$-$C_{36}$
ou sur un alcoyl-,cycloalcoyl- ou alcoylaryl-glycidyléther de formule :

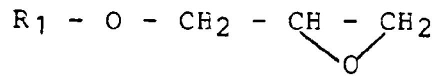

où $R_1$ a les significations ci-dessus indiquées.

Les produits d'addition sont soumis à l'hydrolyse.

Les mercaptans de formule (I) dans lesquels $R_2$ désigne un radical alcoyle ou alcoxyméthyle peuvent être préparés, par exemple, à partir d'alcools à deux chaînes grasses, eux-mêmes préparés, par exemple, par réaction d'un époxyde gras sur un alcool gras. Ces composés sont décrits dans le brevet français 2 465 780 et dans le brevet US 4 666 711.

L'invention a également pour objet les produits non ioniques polyhydroxylés, de formule (V) qui sont solubles ou dispersibles dans l'eau selon la structure du composé de départ de formule (I) et le nombre de motifs hydrophiles.

Ces produits de formule (V) présentent des propriétés moussantes, émulsionnantes ou dispersantes. Ils peuvent d'autre part être autoémulsionnables, c'est-à-dire se disperser facilement dans l'eau en donnant des dispersions laiteuses stables qui peuvent se présenter sous forme de microdispersions vésiculaires susceptibles éventuellement de véhiculer des substances actives.

Les nouveaux produits non-ioniques de l'invention ont des propriétés de surface et peuvent être utilisés comme tensio-actifs dans des compositions cosmétiques pour les soins de la peau et des cheveux telles que les compositions moussantes, comme les shampooings, les bains moussants, les compositions nettoyantes, des laits ou des crèmes pour le visage ou pour le corps, des compositions tinctoriales ou de coloration de la peau, des compositions solaires, des compositions adoucissantes ou démêlantes.

Les tensio-actifs de l'invention peuvent être aisément associés aux différents constituants généralement présents dans les compositions cosmétiques ou pharmaceutiques.

Ils peuvent être associés à d'autres tensio-actifs non-ioniques ou ioniques, à des polymères naturels ou synthétiques, à des huiles ou des cires minérales, animales ou végétales ou à des dérivés de polysiloxanes.

L'invention a également pour objet des compositions contenant les composés tensio-actifs de formule (V).

Parmi ces compositions, il faut citer les compositions cosmétiques, les compositions pharmaceutiques et les compositions utilisées pour les textiles.

Les compositions cosmétiques conviennent plus particulièrement au traitement des cheveux, de la peau et du cuir chevelu.

Ces compositions peuvent comprendre des solvants et en particulier des solvants alcooliques, des propulseurs, des épaississants, des conservateurs, des filtres solaires, des colorants, des parfums, des émulsionnants, des stérols, des agents hydratants, des produits actifs pour le traitement des affections de la peau ou du cuir chevelu, pour la repousse des cheveux, des tensio-actifs non-ioniques ou ioniques, des polymères naturels ou synthétiques, des huiles ou des cires minérales, animales ou végétales, des dérivés de polysiloxane ainsi que les divers adjuvants habituellement utilisés dans les compositions cosmétiques et pharmaceutiques.

Ces compositions peuvent se présenter sous des formes diverses, notamment sous forme de solution, de lotion hydroalcoolique, d'émulsion huile-dans-l'eau ou eau-dans-l'huile, de microémulsion, de microdispersion de vésicules lipidiques renfermant facultativement des produits actifs, éventuellement en présence d'huile, de gel, de pain ou de produits à pulvériser.

L'invention a également pour objet l'utilisation des compositions renfermant un composé de formule (V), plus particulièrement dans les domaines cosmétiques, pharmaceutiques et textiles.

L'invention sera mieux comprise à l'aide des exemples non limitatifs ci-après.

EXEMPLE 1

Préparation d'un mélange de composés de formule (V), dans laquelle :

$R_1 = C_{10}H_{21}$; $R_2 = H$; m = t = u = w = zéro; v = 1; a = 1,5.

A 20,2 g (0,1 mole) de dodécylmercaptan, on ajoute 0,9 g de méthylate de sodium à 5,6 meq/g, puis sous atmosphère d'azote, 18,8 g (0,1 mole) d'isopropylidène-1,2 époxypropyl-3 glycérol (composé de formule (II)) goutte à goutte à la température de 75°C. La durée de l'introduction est de 25 minutes.

On chauffe progressivement jusqu'à 150°C en éliminant le méthanol, puis on ajoute encore 9,4 g (0,05 mole) de composé de formule (II) en l'espace de 20 minutes et on maintient le chauffage pendant 2 heures.

On reprend la masse réactionnelle avec 30 ml d'isopropanol et 3 ml d'eau en présence de 1 ml d'acide chlorhydrique 11,6 N.

Après une nuit à la température ambiante, on neutralise avec la quantité équivalente de NaOH, puis on

évapore les solvants par chauffage sous pression réduite.

Le résidu est repris à l'eau et la solution est ajustée à 30% de matières actives. La solution est limpide et de couleur brune.

Le point de trouble d'une solution à 0,5% de matières actives dans l'eau est de 70°C.

EXEMPLE 2

Préparation d'un mélange de composés de formule (V), dans laquelle :

$R_1 = C_{10}H_{21}$; $R_2$ = H; m = t = u = zéro; v = w = 1; a = 1,5.

A 100 g de solution aqueuse à 30% de matières actives, préparée à l'exemple 1 et contenant 0,07 équivalent de thioéther, on ajoute 6,9 g (0,07 équivalent) d'eau oxygénée à 10,2 moles/litre à la température de 30°C en 30 minutes.

On maintient à cette température pendant 3 heures 30 minutes.

On obtient une solution limpide de couleur brune.

Le point de trouble d'une solution aqueuse à 0,5% est supérieur à 100°C.

Le point de trouble mesuré dans une solution aqueuse à 10% de chlorure de sodium est également supérieur à 100°C.

EXEMPLE 3

Préparation d'un mélange de composés de formule (V), dans laquelle :

$R_1 = C_{17}H_{35}$; m = t = u = v = w = zéro; a = 2.

A 20 g (0,07 mole) d'octadécanethiol on ajoute 0,62 g de solution méthanolique de méthylate de sodium à 5,65 meq/g, puis à la température de 75-80°C, en 30 minutes, 13,16 g (0,07 mole) d'époxyde de formule (II) (isopropylidène-1,2 epoxypropyl-3 glycérol) sous atmosphère d'azote. On élève ensuite la température à 150°C en éliminant le méthanol, puis on ajoute encore 13,16 g (0,07 mole) d'époxyde (II) en 30 minutes.

On maintient le chauffage et l'agitation pendant encore 30 minutes.

La masse réactionnelle est reprise avec 30 ml d'isopropanol et 10 ml d'eau en présence de 0,7 ml d'acide chlorhydrique concentré. La solution est chauffée à 40°C pendant 5 heures. Le catalyseur est neutralisé avec la quantité équivalente de soude et les solvants sont éliminés sous pression réduite.

On obtient ainsi une pâte dure de couleur brun clair, dispersible dans l'eau en donnant une dispersion visqueuse et translucide.

EXEMPLES D'APPLICATION

EXEMPLE A - Shampoing

| | | |
|---|---|---|
| - Composés de l'exemple 1 | | 10,0 g MA |
| - Dodécanediol-1,2 | | 0,7 g MA |
| - Parfum, conservateur | qs | |
| - Eau | qsp | 100,0 g |

Le pH est adjusté à 7,0 par de la soude.

MA = matière active

EXEMPLE B - Bain moussant

| | | |
|---|---|---|
| - Composés de l'exemple 1 | | 10,0 g MA |
| - Lauryl éther sulfate de sodium oxyéthyléné avec 2,2 moles d'oxyde d'éthylène | | 10,0 g MA |
| - Acide éthylène diamine tétracétique | | 0,2 g |
| - Eau | qsp | 100,0 g |

Le pH est adjusté à 7,0 par de la soude.

EXEMPLE C - Lait adoucissant pour le corps

| | | |
|---|---|---|
| - Composés de l'exemple 3 | | 10,0 g MA |
| - Huile de vaseline | | 40,0 g MA |
| - Eau | qsp | 100,0 g |

Le mélange de composés de l'exemple 3 est solubilisé dans l'huile de vaseline en chauffant jusqu'à 80-90°C. On refroidit vers 60°C et on ajoute l'eau, préalablement chauffée à 60°C, tout en agitant vigoureusement.

On obtient une émulsion fine et stable.

## Revendications

### Revendications pour les Etats contractants suivants : AT, BE, CH, DE, DK, FR, GB, IT, LI, NL, SE

**1.** Procédé de préparation de produits tensio-actifs non-ioniques à partir de l'isopropylidène-1,2 époxypropyl-3 glycérol, comprenant dans une première étape la réaction d'un composé de formule (I) :

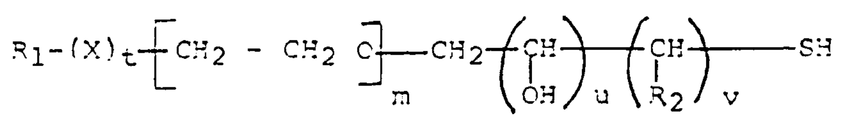

(I)

dans laquelle :

$R_1$ désigne un radical hydrocarboné aliphatique, cycloaliphatique ou alkylarylique en $C_8$ à $C_{36}$,

$R_2$ désigne un radical alcoyle ou alcoxyméthyle en $C_8$ à $C_{21}$ ou un atome d'hydrogène,

X désigne -O- ou -S- ,

m désigne zéro ou un nombre entier ou décimal de 1 à 20,

t, u et v désignent zéro ou le nombre 1,

quand u = 1, v est obligatoirement égal à 1 et $R_2$ = H, et

quand v = 1 et $R_2$ est différent de H, alors u est égal à zéro, la somme des atomes de carbone dans $R_1$ et $R_2$ est au moins de 8 et au plus de 36,

avec l'isopropylidène-1,2 époxypropyl-3 glycérol de formule :

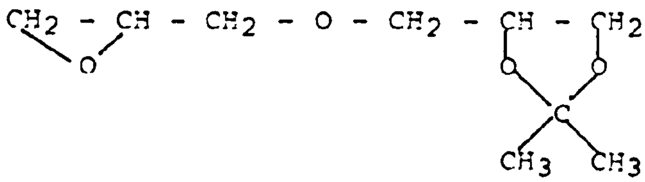 (II)

et dans une deuxième étape, l'hydrolyse des produits de la condensation, la fonction mercaptan pouvant être oxydée en sulfoxyde avec de l'eau oxygénée, caractérisé par le fait qu'on fait réagir plus d'une mole d'époxyde par mole de mercaptan :

$(n > 1)$

et que la quantité molaire d'époxyde de formule (II) équivalente à la quantité molaire de mercaptan, est ajoutée à la température comprise entre 40 et 90°C et que la quantité restante d'époxyde est ajoutée progressivement en l'espace de 1 à 3 heures, à une température comprise entre 130 et 160°C, après avoir éliminé le solvent éventuel.

**2.** Procédé selon la revendication 1, caractérisé par le fait que la polyaddition de l'époxyde de formule (II) sur le mercaptan de formule (I) est réalisé en présence d'un catalyseur basique comme le méthylate ou l'éthylate de sodium ou de potassium ou le t-butylate de potassium, à une température comprise entre 40 et 160°C.

**3.** Procédé selon la revendication 1 ou 2, caractérisé par le fait que la réaction est mise en oeuvre en présence d'un solvant polaire.

**4.** Procédé selon la revendication 3, caractérisé par le fait qu'on utilise comme solvant polaire un alcool en $C_1$-$C_4$.

**5.** Procédé selon la revendication 1 ou 2, caractérisé par le fait que la réaction est mise en oeuvre dans un solvant non polaire comme le toluène.

**6.** Produits tensio-actifs non-ioniques de formule :

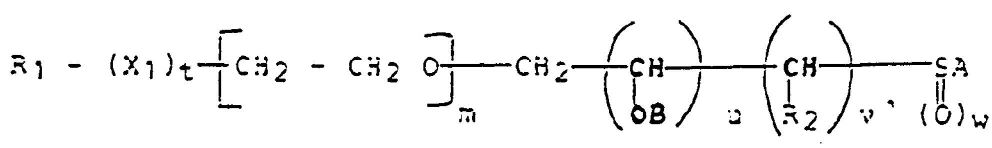 (V)

dans laquelle $R_1$ désigne un radical hydrocarboné aliphatique, cycloaliphatique, ou alkylarylique en $C_8$ à $C_{36}$,

$R_2$ désigne un radical alcoyle ou alcoxyméthyle en $C_8$ à $C_{21}$ ou un atome d'hydrogène,
la somme des atomes de carbone dans $R_1$ et $R_2$ est au moins de 8 et au plus de 36,
$X_1$ désigne -O-, -S- ou S=O quand w=1
m désigne zéro ou un nombre entier ou décimal de 1 à 20,
t, u et v désignent zéro ou le nombre 1,
quand u = 1, v est obligatoirement égal à 1 et $R_2$ = H, et
quand v = 1 et $R_2$ est différent de H, alors u est égal à zéro,
w désigne O ou 1,
A désigne l'enchaînement

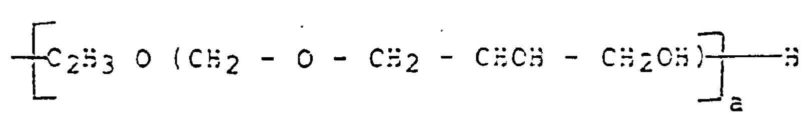

et B désigne l'enchaînement

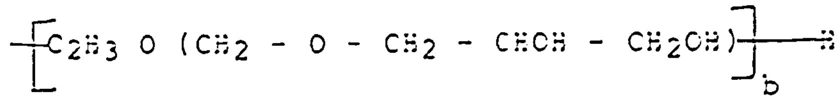

a désigne un nombre entier ou décimal de 1,5 à 8,
b désigne zéro ou un nombre entier ou décimal tel que a+b = n,
n désignant un nombre entier ou décimal de 1,5 à 8.

**7.** Produits tensio-actifs non-ioniques de formule :

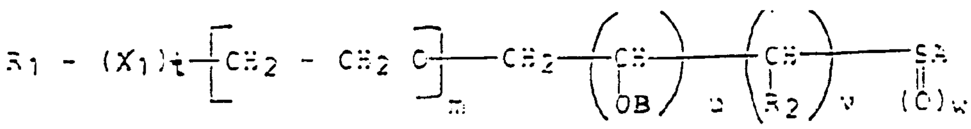 (V)

dans laquelle $R_1$ désigne un radical hydrocarboné aliphatique, cycloaliphatique, ou alkylarylique en $C_8$ à $C_{36}$,

$R_2$ désigne un radical alcoyle ou alcoxyméthyle en $C_8$ à $C_{21}$ ou un atome d'hydrogène,
la somme des atomes de carbone dans $R_1$ et $R_2$ est au moins de 8 et au plus de 36,
$X_1$ désigne -O-, -S- ou S=O quand w=1
m désigne zéro ou un nombre entier ou décimal de 1 à 20,
u désigne zéro,
t et v désignent zéro ou le nombre 1,
w désigne O ou 1,
A désigne l'enchaînement

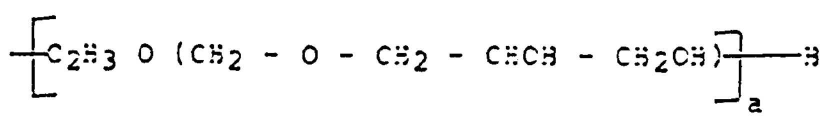

a désigne un nombre entier ou décimal de 1,5 à 8.

**8.** Produits tensio-actifs non-ioniques selon la revendication 7, caractérisé par le fait que $R_1$, $R_2$, t, m, v, w et a ont les significations ci-après :

(i) $R_1 = C_{10}H_{21}$; $R_2$ = H; m=t=w=zéro; v=1; a=1,5
(ii) $R_1 = C_{10}H_{21}$; $R_2$ = H; m=t=zéro; v=w=1; a=1,5
(iii) $R_1 = C_{17}H_{35}$; m=t=v=w=zéro; a=2.

**9.** Composition comprenant un ou plusieurs composés de formule (V) préparés selon les revendications 6 à 8, dans un véhicule approprié.

**10.** Composition cosmétique ou pharmaceutique, caractérisée par le fait qu'elle comprend un ou plusieurs composés de formule (V) selon les revendications 6 à 8 dans un véhicule approprié.

**11.** Composition selon la revendication 9, destinée à l'industrie textile.

**12.** Composition selon la revendication 9 ou 10, caractérisée par le fait qu'elle renferme également un ou plusieurs produits choisis parmi les tensio-actifs non-ioniques, les tensio-actifs ioniques, les polymères naturels ou synthétiques, les huiles et les cires minérales, animales ou végétales, les dérivés de polysiloxanes, les solvants, les propulseurs, les épaississants, les conservateurs, les filtres solaires, les colorants, les parfums, les agents émulsionnants, les stérols, les produits hydratants, les produits actifs pour le traitement des affections de la peau ou du cuir chevelu, les produits pour la repousse des cheveux.

**13.** Composition selon les revendications 9, 10 et 12, caractérisée par le fait qu'elle se présente sous forme de solution aqueuse, de lotion hydroalcoolique, d'émulsion huile-dans-eau ou eau-dans-huile, de microémulsion, de microdispersions de vésicules lipidiques contenant ou ne contenant pas de produits actifs,

de gel, de pain ou de produits à pulvériser.

**Revendications pour l'Etat contractant suivant : ES**

1. Procédé de préparation de produits tensio-actifs non-ioniques à partir de l'isopropylidène- 1,2 époxypropyl-3 glycérol, comprenant dans une première étape la réaction d'un composé de formule (I) :

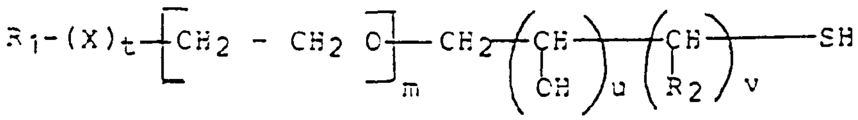 (I)

dans laquelle :
$R_1$ désigne un radical hydrocarboné aliphatique, cycloaliphatique ou alkylarylique en $C_8$ à $C_{36}$,
$R_2$ désigne un radical alcoyle ou alcoxyméthyle en $C_8$ à $C_{21}$ ou un atome d'hydrogène,
X désigne -0- ou -S- ,
m désigne zéro ou un nombre entier ou décimal de 1 à 20,
t, u et v désignent zéro ou le nombre 1,
quand u = 1, v est obligatoirement égal à 1 et $R_2$ = H, et
quand v = 1 et $R_2$ est différent de H, alors u est égal à zéro, la somme des atomes de carbone dans $R_1$ et $R_2$ est au moins de 8 et au plus de 36,
avec l'isopropylidène- 1,2 époxypropyl-3 glycérol de formule :

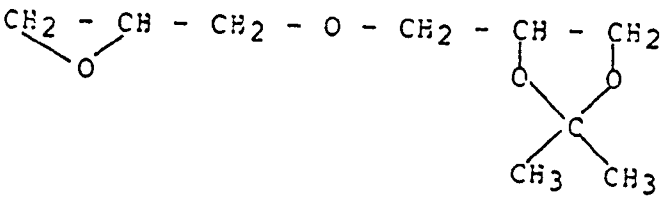 (II)

et dans une deuxième étape, l'hydrolyse des produits de la condensation , la fonction mercaptan pouvant être oxydée en une troisième étape en sulfoxyde, avec de l'eau oxygénée, caractérisé par le fait qu'on fait réagir plus d'une mole d'époxyde par mole de mercaptan (n > 1) et que la quantité molaire d'époxyde de formule (I) équivalente à la quantité molaire de mercaptan, est ajoutée à la température comprise entre 40° et 90°C et que la quantité restante d'époxyde est ajoutée progressivement en l'espace de 1 à 3 heures, à une température comprise entre 130 et 160°C, après avoir éliminé le solvant éventuel.

2. Procédé selon la revendication 1, caractérisé par le fait que la polyaddition de l'époxyde de formule (II) sur le mercaptan de formule (I) est réalisée en présence d'un catalyseur basique comme le méthylate ou l'éthylate de sodium ou de potassium ou le t-butylate de potassium, à une température comprise entre 40 et 160°C

3. Procédé selon la revendication 1, caractérisé par le fait que la première addition d'époxyde est mise en oeuvre en présence d'un solvant polaire.

4. Procédé selon la revendication 3, caractérisé par le fait qu'on utilise comme solvant polaire un alcool en $C_1$-$C_4$.

5. Procédé selon la revendication 1, caractérisé par le fait que la première addition d'époxyde est mise en oeuvre dans un solvant non polaire comme le toluène.

6. Procédé de préparation de produits tensio-actifs non-ioniques selon les revendications 1 à 5, caractérisé par le fait qu'on prépare les composés de formule (V) :

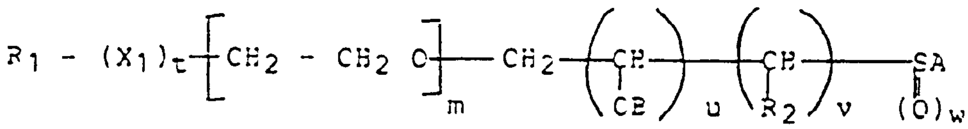 (V)

dans laquelle $R_1$ désigne un radical hydrocarboné aliphatique, cycloaliphatique, ou alkylarylique en $C_8$ à $C_{36}$,

$R_2$ désigne un radical alcoyle ou alcoxyméthyle en $C_8$ à $C_{21}$ ou un atome d'hydrogène,
la somme des atomes de carbone dans $R_1$ et $R_2$ est au moins de 8 et au plus de 36,
$X_1$ désigne -0-, -S- ou S=0 quand w=1
m désigne zéro ou un nombre entier ou décimal de 1 à 20,
t, u et v désignent zéro ou le nombre 1,
quand u = 1, v est obligatoirement égal à 1 et $R_2$ = H, et
quand v = 1 et $R_2$ est différent de H, alors u est égal à zéro,
w désigne O ou 1,
A désigne l'enchaînement

$$-\left[C_2H_3\ O\ (CH_2 - O - CH_2 - CHCH - CH_2CH)\right]_a - H$$

et B désigne l'enchaînement

$$-\left[C_2H_3\ O\ (CH_2 - O - CH_2 - CHOH - CH_2OH)\right]_b - H$$

a désigne un nombre entier ou décimal de 1,5 à 8,
b désigne zéro ou un nombre entier ou décimal tel que a+b = n,
n désigne un nombre entier ou décimal de 1,5 à 8.

7. Procédé de préparation de produits tensio-actifs non ioniques de formule (V) selon les revendications 1 à 5, caractérisé par le fait qu'on prépare des composés de formule :

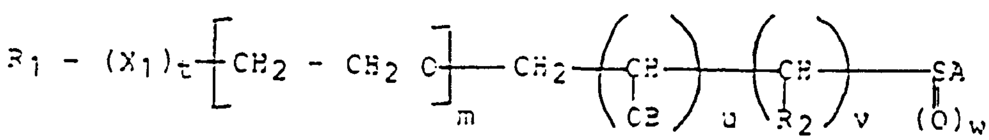 (V),

dans laquelle $R_1$ désigne un radical hydrocarboné aliphatique, cycloaliphatique, ou alkylarylique en $C_8$ à $C_{36}$,

$R_2$ désigne un radical alcoyle ou alcoxyméthyle en $C_8$ à $C_{21}$ ou un atome d'hydrogène,
la somme des atomes de carbone dans $R_1$ et $R_2$ est au moins de 8 et au plus de 36,
$X_1$ désigne -O-, -S- ou S=O quand w=1
m désigne zéro ou un nombre entier ou décimal de 1 à 20,
u désigne zéro,
t et v désignent zéro ou le nombre 1,
w désigne O ou 1,
A désigne l'enchaînement

$$-\left[C_2H_3O(CH_2-O-CH_2-CHOH-CH_2OH)\right]_a-H$$

a désigne un nombre entier ou décimal de 1,5 à 8.

**8.** Procédé de préparation de produits tensio-actifs non-ioniques de formule (V), selon la revendication 7, caractérisé par le fait que $R_1$, $R_2$, t, m, v, w et a ont les significations ci-après :
(i) $R_1 = C_{10}H_{21}$; $R_2$ = H; m=t=w=zéro; v=1; a=1,5
(ii) $R_1 = C_{10}H_{21}$; $R_2$ = H; m=t=zéro; v=w=1; a=1,5
(iii) $R_1 = C_{17}H_{35}$; m=t=v=w=zéro; a=2.

**9.** Composition comprenant un ou plusieurs composés de formule (V) préparés selon les revendications 6 à 8, dans un véhicule approprié.

**10.** Composition cosmétique caractérisée par le fait qu'elle comprend un ou plusieurs composés de formule (V) dans un véhicule approprié.

**11.** Composition selon la revendication 9, destinée à l'industrie textile.

**12.** Composition selon la revendication 9 ou 10, caractérisée par le fait qu'elle renferme également un ou plusieurs produits choisis parmi les tensio-actifs non-ioniques, les tensio-actifs ioniques, les polymères naturels et synthétiques, les huiles et les cires minérales, animales ou végétales, les dérivés de polysiloxanes, les solvants, les propulseurs, les épaississants, les conservateurs, les filtres solaires, les colorants, les parfums, les agents émulsionnants, les stérols, les produits hydratants, les produits actifs pour le traitement des affections de la peau ou du cuir chevelu, les produits pour la repousse des cheveux.

**13.** Composition selon les revendications 10 et 12, caractérisée par le fait qu'elle se présente sous forme de solution aqueuse, de lotion hydroalcoolique, d'émulsion huile-dans-l'eau ou eau-dans-l'huile, de microémulsion, de microdispersions de vésicules lipidiques contenant ou ne contenant pas de produits actifs, de gel, de pain ou de produits à pulvériser.

## Patentansprüche

## Patentansprüche für folgende Vertragsstaaten : AT, BE, CH, DE, DK, FR, GB, IT, LI, NL, SE

**1.** Verfahren zur Herstellung von nichtionischen grenzflächenaktiven Stoffen aus 1,2-Isopropyliden-3-epoxypropyl-glycerin, umfassend in einer ersten Stufe die Umsetzung einer Verbindung der Formel (I):

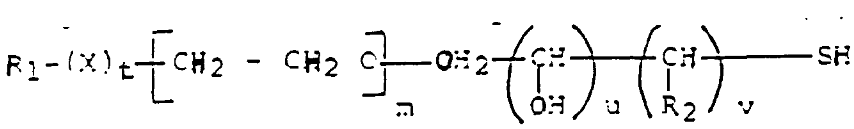

(I)

worin bedeuten:
$R_1$ einen aliphatischen, cycloaliphatischen oder Arylalkyl-Kohlenwasserstoffrest mit $C_8$-$C_{36}$
$R_2$ einen Alkyl- oder Alkoxymethylrest mit $C_8$-$C_{21}$ oder ein Wasserstoffatom,
X -O- oder -S-
m Null oder eine ganze oder Dezimalzahl von 1 bis 20,
t, u und v Null oder die Zahl 1,
und wenn u = 1, ist v zwingend = 1 und $R_2$ = H und
wenn v = 1 und $R_2$ von H verschieden ist, dann ist u gleich Null, und die Summe der Kohlenstoffatome in $R_1$ und $R_2$ beträgt mindestens 8 und höchstens 36,
mit 1,2-Isopropyliden-3-epoxypropyl-glycerin der Formel:

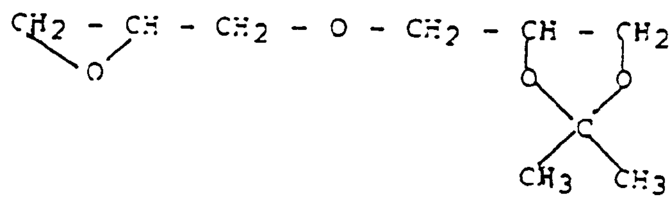 (II)

und in einer zweiten Stufe die Hydrolyse der Kondensationsprodukte, und wobei die Mercaptangruppe mit Wasserstoffperoxid oxidiert werden kann, dadurch gekennzeichnet, daß man mehr als 1 Mol Epoxid pro Mol Mercaptan umsetzt:
( $n > 1$ )
und dadurch, daß die molare Menge des Epoxids der Formel (II), die der molaren Menge an Mercaptan equivalent ist, bei einer Temperatur zwischen 40 und 90°C zugegeben wird, und die restliche Menge an Epoxid allmählich während eines Zeitraums von 1 bis 3 Stunden bei einer Temperatur zwischen 130 und 160°C zugegeben wird, und man danach gegebenenfalls das Lösungsmittel entfernt.

2. Verfahren nach Anspruch 1, dadurch gekennzeichnet, daß die Polyaddition des Epoxids der Formel (II) an das Mercaptan der Formel (I) in Gegenwart eines basischen Katalysators, wie Natrium- oder Kaliummethylat oder - ethylat, oder Kalium-t-butylat, bei einer Temperatur zwischen 40 und 160°C durchgeführt wird.

3. Verfahren nach Anspruch 1 oder 2, dadurch gekennzeichnet, daß die Umsetzung in Gegenwart eines polaren Lösungsmittels durchgeführt wird.

4. Verfahren nach Anspruch 3, dadurch gekennzeichnet, daß man als polares Lösungsmittel einen $C_1$-$C_4$-Alkohol verwendet.

5. Verfahren nach Anspruch 1 oder 2, dadurch gekennzeichnet, daß die Reaktion in einem nicht-polaren Lösungsmittel, wie Toluol, durchgeführt wird.

6. Nichtionische grenzflächenaktive Stoffe der Formel:

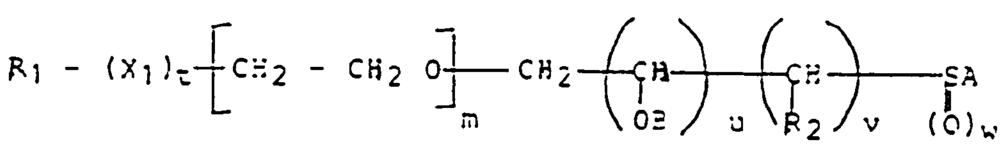 (V)

worin $R_1$ einen aliphatischen, cycloaliphatischen oder Alkylaryl-Kohlenwasserstoffrest mit $C_8$-$C_{36}$, bedeutet,
$R_2$ ein Alkyl- oder Alkoxymethylradikal mit $C_8$-$C_{21}$ oder ein Wasserstoffatom,
die Summe der Kohlenstoffatome in $R_1$ und $R_2$ mindestens 8 und höchstens 36 beträgt,
$X_1$ -O-, -S- oder S = O, wenn w = 1, bedeutet,
m Null oder eine ganze oder Dezimalzahl von 1 bis 20 bedeutet,
t, u und v Null oder die Zahl 1 bedeuten,
und wenn u = 1, dann ist v zwingend gleich 1, und $R_2$ = H,
und wenn v = 1 und $R_2$ von H verschieden ist, dann ist u gleich Null,
w 0 oder 1 bedeutet,
A die Kette

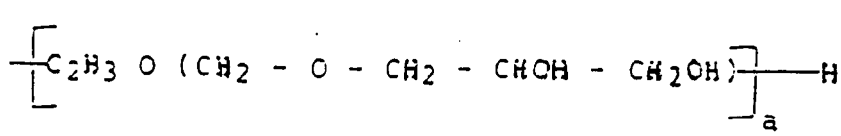

bedeutet

und B die Kette

$$-\left[C_2H_3O(CH_2-O-CH_2-CHOH-CH_2OH)\right]_b-H$$

bedeutet,
a eine ganze Zahl oder eine Dezimalzahl von 1.5 bis 8 bedeutet,
b Null oder eine ganze oder Dezimalzahl bedeutet, wobei a + b = n,
n eine ganze oder Dezimalzahl von 1.5 bis 8 bedeutet.

**7.** Nichtionische grenzflächenaktive Stoffe der Formel:

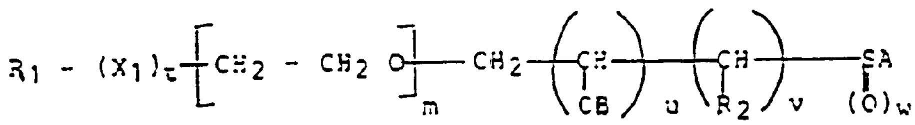

(V)

worin $R_1$ einen aliphatischen, cycloaliphtischen oder Alkylaryl-Kohlenwasserstoffrest mit $C_8$-$C_{36}$ bedeutet,
$R_2$ einen Alkyl- oder Alkoxymethylrest mit $C_8$-$C_{21}$ oder ein Wasserstoffatom bedeutet,
die Summe der Kohlenstoffatome in $R_1$ und $R_2$ mindestens 8 und höchstens 36 ist,
X, -O-, -S- oder S = O, wenn w = 1, bedeutet,
m Null oder eine ganze oder Dezimalzahl von 1 bis 20 bedeutet,
u Null bedeutet,
t und v Null oder die Zahl 1 bedeuten,
w Null oder 1 bedeutet,
A die Kette

$$-\left[C_2H_3O(CH_2-O-CH_2-CHOH-CH_2OH)\right]_a-H$$

worin a eine ganze oder Dezimalzahl von 1.5 bis 8 bedeutet.

**8.** Nichtionische grenzflächenaktive Stoffe gemäß Anspruch 7, dadurch gekennzeichnet, daß $R_1$, $R_2$, t, m, v, w und a die folgenden Bedeutungen besitzen:
(i) $R_1=C_{10}H_{21}$; $R_2=H$; m=t=w=Null; v=1; a=1.5
(ii) $R_1=C_{10}H_{21}$; $R_2=H$; m=t=Null; v=w=1; a=1.5
(iii) $R_1=C_{17}H_{35}$; m=t=v=w=Null; a=2

**9.** Zusammensetzung umfassend eine oder mehrere Verbindungen der Formel (V) gemäß den Ansprüchen 6 bis 8 in einem geeigneten Träger.

**10.** Kosmetische oder pharmazeutische Zusammensetzung, dadurch gekennzeichnet, daß sie eine oder mehrere Verbindungen der Formel (V) gemäß den Ansprüchen 6 bis 8 in einem geeigneten Träger enthält.

**11.** Zusammensetzung nach Anspruch 9 für die Textilindustrie.

**12.** Zusammensetzung gemäß Anspruch 9 oder 10, dadurch gekennzeichnet, daß sie auch eine oder mehrere Stoffe enthält ausgewählt aus den nichtionischen grenzflächenaktiven Stoffen, den ionischen grenzflächenaktiven Stoffen, den natürlichen und synthetischen Polymeren, den den mineralischen, tierischen oder pflanzlichen Ölen oder Wachsen, den Polysiloxanderivaten, den Lösungsmitteln, den Treibmitteln, den Verdickungsmitteln, den Konservierungsmitteln, den Sonnenfiltern, den Farbstoffen, den Parfüms, den Emulgiermitteln, den Sterolen, den wasseranlagernden Mitteln, den Wirkstoffen zur Behandlung von Erkrankungen der Haut oder der Kopfhaut, den Haarwuchsmitteln.

13. Zusammensetzung gemäß den Ansprüchen 9, 10 und 12, dadurch gekennzeichnet, daß sie in Form einer wässerigen Lösung, einer wässerig-alkoholischen Lotion, einer Öl-in-Wasser -oder-Wasser-in-Öl-Emulsion, einer Mikroemulsion, von Mikrodispersionen von Lipidvesikeln, die einen Wirkstoff enthalten oder nicht enthalten, eines Gels, einer Masse oder von pulverförmigen Produkten vorliegt.

**Patentansprüche für folgenden Vertragsstaat : ES**

1. Verfahren zur Herstellung von nichtionischen grenzflächenaktiven Stoffen aus 1,2-Isopropyliden-3-epoxypropylglycerin, umfassend in einer ersten Stufe die Umsetzung einer Verbindung der Formel (I):

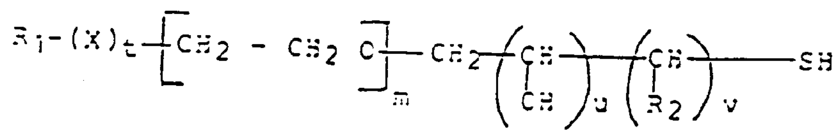 (I)

worin bedeuten:
$R_1$ einen aliphatischen, cycloaliphatischen oder Arylalkyl-Kohlenwasserstoffrest mit $C_8$-$C_{36}$
$R_2$ einen Alkyl- oder Alkoxymethylrest mit $C_8$-$C_{21}$ oder ein Wasserstoffatom,
X -O- oder -S-
m Null oder eine ganze oder Dezimalzahl von 1 bis 20,
t, u und v Null oder die Zahl 1,
und wenn u = 1, ist v zwingend = 1 und $R_2$ = H und
wenn v = 1 und $R_2$ von H verschieden ist, dann ist u gleich Null, und die Summe der Kohlenstoffatome in $R_1$ und $R_2$ beträgt mindestens 8 und höchstens 36,
mit 1,2-Isopropyliden-3-epoxypropyl-glycerin der Formel:

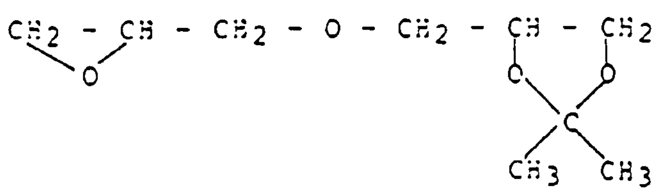 (II)

und in einer zweiten Stufe die Hydrolyse der Kondensationsprodukte, und wobei die Mercaptangruppe in einer dritten Stufe mit Wasserstoffperoxid oxidiert werden kann, dadurch gekennzeichnet, daß man mehr als 1 Mol Epoxid pro Mol Mercaptan umsetzt:
( n > 1 )
und dadurch, daß die molare Menge des Epoxids der Formel (II), die der molaren Menge an Mercaptan equivalent ist, bei einer Temperatur zwischen 40 und 90°C zugegeben wird, und die restliche Menge an Epoxid allmählich während eines Zeitraums von 1 bis 3 Stunden bei einer Temperatur zwischen 130 und 160°C zugegeben wird, und man danach gegebenenfalls das Lösungsmittel entfernt.

2. Verfahren nach Anspruch 1, dadurch gekennzeichnet, daß die Polyaddition des Epoxids der Formel (II) an das Mercaptan der Formel (I) in Gegenwart eines basischen Katalysators, wie Natrium- oder Kaliummethylat oder - ethylat, oder Kalium-t-butylat, bei einer Temperatur zwischen 40 und 160°C durchgeführt wird.

3. Verfahren nach Anspruch 1 oder 2, dadurch gekennzeichnet, daß die erste Addition des Epoxids in Gegenwart eines polaren Lösungsmittels durchgeführt wird.

4. Verfahren nach Anspruch 3, dadurch gekennzeichnet, daß man als polares Lösungsmittel einen $C_1$-$C_4$-Alkohol verwendet.

5. Verfahren nach Anspruch 1, dadurch gekennzeichnet, daß die erste Addition des Epoxids in einem nichtpolaren Lösungsmittel, wie Toluol, durchgeführt wird.

6. Verfahren zur Herstellung von nichtionischen grenzflächenaktiven Stoffen gemäß den Ansprüchen 1 bis 5, dadurch gekennzeichnet, daß man die Verbindungen der Formel (V) herstellt:

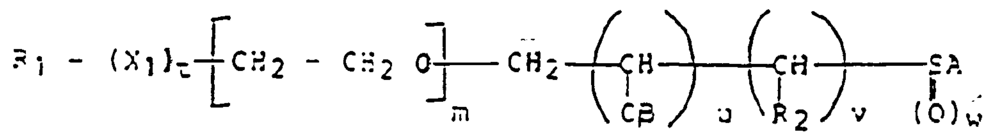 (V)

worin $R_1$ einen aliphatischen, cycloaliphatischen oder Alkylaryl-Kohlenwasserstoffrest mit $C_8$-$C_{36}$, bedeutet,
$R_2$ ein Alkyl- oder Alkoxymethylradikal mit $C_8$-$C_{21}$ oder ein Wasserstoffatom,
die Summe der Kohlenstoffatome in $R_1$ und $R_2$ mindestens 8 und höchstens 36 beträgt,
$X_1$ -O-, -S- oder S = O, wenn w = 1, bedeutet,
m Null oder eine ganze oder Dezimalzahl von 1 bis 20 bedeutet,
t, u und v Null oder die Zahl 1 bedeuten,
und wenn u = 1, dann ist v zwingend gleich 1, und $R_2$ = H,
und wenn v = 1 und $R_2$ von H verschieden ist, dann ist u gleich Null,
w 0 oder 1 bedeutet,
A die Kette

$$-\left[C_2H_3O(CH_2-O-CH_2-CHOH-CH_2OH)\right]_a-H$$

bedeutet
und B die Kette

$$-\left[C_2H_3O(CH_2-O-CH_2-CHOH-CH_2OH)\right]_b-H$$

bedeutet,
a eine ganze Zahl oder eine Dezimalzahl von 1.5 bis 8 bedeutet,
b Null oder eine ganze oder Dezimalzahl bedeutet, wobei a + b = n,
n eine ganze oder Dezimalzahl von 1.5 bis 8 bedeutet.

7. Verfahren zur Herstellung von nichtionischen grenzflächenaktiven Stoffen der Formel (V) gemäß den Ansprüchen 1 bis 5, dadurch gekennzeichnet, daß man die Verbindungen der Formel herstellt:

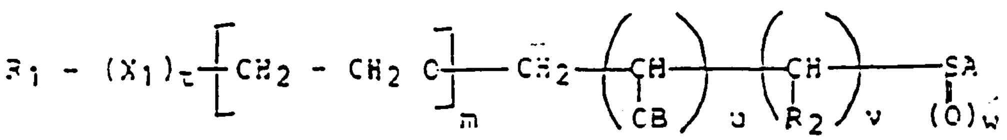 (V)

worin $R_1$ einen aliphatischen, cycloaliphtischen oder Alkylaryl-Kohlenwasserstoffrest mit $C_8$-$C_{36}$ bedeutet,
$R_2$ einen Alkyl- oder Alkoxymethylrest mit $C_8$-$C_{21}$ oder ein Wasserstoffatom bedeutet,
die Summe der Kohlenstoffatome in $R_1$ und $R_2$ mindestens 8 und höchstens 36 ist,
X, -O-, -S- oder S = O, wenn w = 1, bedeutet,
m Null oder eine ganze oder Dezimalzahl von 1 bis 20 bedeutet,
u Null bedeutet,
t und v Null oder die Zahl 1 bedeuten,
w Null oder 1 bedeutet,

A die Kette

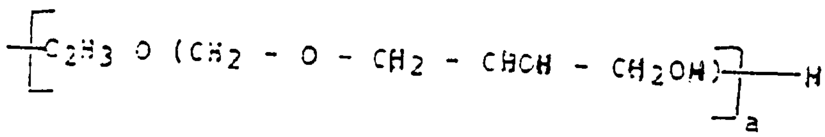

worin a eine ganze oder Dezimalzahl von 1.5 bis 8 bedeutet.

**8.** Verfahren zur Herstellung von nichtionischen grenzflächenaktiven Stoffen der Formel (V) gemäß Anspruch 7, dadurch gekennzeichnet, daß $R_1$, $R_2$, t, m, v, w und a die folgenden Bedeutungen besitzen:
(i) $R_1=C_{10}H_{21}$; $R_2=H$; m=t=w=Null; v=1; a=1.5
(ii) $R_1=C_{10}H_{21}$; $R_2=H$; m=t=Null; v=w=1; a=1.5
(iii) $R_1=C_{17}H_{35}$; m=t=v=w=Null; a=2

**9.** Zusammensetzung umfassend eine oder mehrere Verbindungen der Formel (V), hergestellt nach den Ansprüchen 6 bis 8, in einem geeigneten Träger.

**10.** Kosmetische Zusammensetzung, dadurch gekennzeichnet, daß sie eine oder mehrere Verbindungen der Formel (V) in einem geeigneten Träger umfaßt.

**11.** Zusammensetzung nach Anspruch 9 für die Textilindustrie.

**12.** Zusammensetzung gemäß Anspruch 9 oder 10, dadurch gekennzeichnet, daß sie auch eine oder mehrere Stoffe enthält ausgewählt aus den nichtionischen grenzflächenaktiven Stoffen, den ionischen grenzflächenaktiven Stoffen, den natürlichen und synthetischen Polymeren, den den mineralischen, tierischen oder pflanzlichen Ölen oder Wachsen, den Polysiloxanderivaten, den Lösungsmitteln, den Treibmitteln, den Verdickungsmitteln, den Konservierungsmitteln, den Sonnenfiltern, den Farbstoffen, den Parfüms, den Emulgiermitteln, den Sterolen, den wasseranlagernden Mitteln, den Wirkstoffen zur Behandlung von Erkrankungen der Haut oder der Kopfhaut, den Haarwuchsmitteln.

**13.** Zusammensetzung gemäß den Ansprüchen 10 und 12, dadurch gekennzeichnet, daß sie in Form einer wässerigen Lösung, einer wässerig-alkoholischen Lotion, einer Öl-in-Wasser-oder -Wasser-in-Öl-Emulsion, einer Mikroemulsion, von Mikrodispersionen von Lipidvesikeln, die einen Wirkstoff enthalten oder nicht enthalten, eines Gels, einer Masse oder von pulverförmigen Produkten vorliegt.

## Claims

### Claims for the following Contracting States : AT, BE, CH, DE, DK, FR, GB, IT, LI, NL, SE

**1.** Process for preparing nonionic surface-active products from 1,2-isopropylidene-3-epoxyropyl-glycerol, comprising, in a first stage, reacting a compound of formula (I):

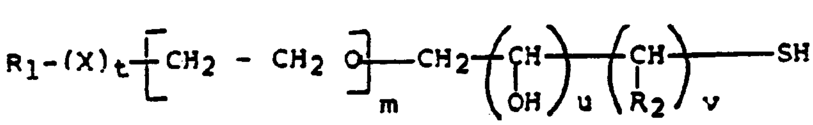 (I)

in which:
$R_1$ denotes an aliphatic, cycloaliphatic or alkylaryl $C_8$ to $C_{36}$ hydrocarbon radical,
$R_2$ denotes a $C_8$ to $C_{21}$ alkyl or alkoxymethyl radical or a hydrogen atom,
X denotes -O- or -S-,
m denotes zero or an integer or decimal from 1 to 20,
t, u and v denote zero or the number 1,
when u = 1, v is necessarily equal to 1 and $R_2$ = H, and
when v = 1 and $R_2$ is different from H, then u is equal to zero, the sum of the carbon atoms in $R_1$ and $R_2$ is not less than 8 and not more than 36,

with 1,2-isopropylidene-3-epoxypropylglycerol of formula:

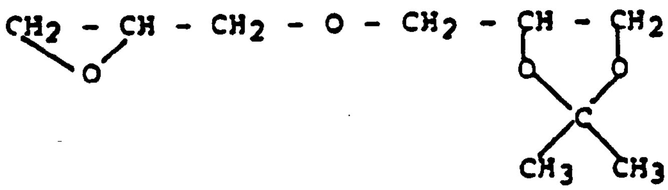

(II)

and, in a second stage, hydrolysing the condensation products, it being possible for the mercaptan functional group to be oxidised to a sulphoxide with hydrogen peroxide, characterised in that more than one mole of epoxide is reacted per mole of mercaptan:

($n > 1$)

and in that the molar amount of epoxide of formula (II), equivalent to the molar amount of mercaptan, is added at a temperature of between 40 and 90°C, and in that the remaining amount of epoxide is progressively added over 1 to 3 hours, at a temperature of between 130 and 160°C, after removing the solvent where appropriate.

2. Process according to Claim 1, characterised in that the polyaddition of the epoxide of formula (II) to the mercaptan of formula (I) is carried out in the presence of a basic catalyst such as sodium or potassium methoxide or ethoxide or potassium t-butoxide, at a temperature of between 40 and 160°C.

3. Process according to Claim 1 or 2, characterised in that the reaction is carried out in the presence of a polar solvent.

4. Process according to Claim 3, characterised in that a $C_1$-$C_4$ alcohol is used as polar solvent.

5. Process according to Claim 1 or 2, characterised in that the reaction is carried out in a nonpolar solvent such as toluene.

6. Nonionic surface-active products of formula:

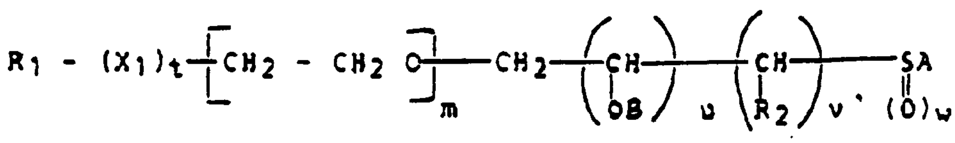

(V)

in which $R_1$ denotes an aliphatic, cycloaliphatic or alkylaryl $C_8$ to $C_{36}$ hydrocarbon radical,
$R_2$ denotes a $C_8$ to $C_{21}$ alkyl or alkoxymethyl radical or a hydrogen atom,
the sum of the carbon atoms in $R_1$ and $R_2$ is not less than 8 and not more than 36,
$X_1$ denotes -O-, -S- or S=O when w=1,
m denotes zero or an integer or decimal from 1 to 20,
t, u and v denote zero or the number 1,
when u = 1, v is necessarily equal to 1 and $R_2$ = H, and
when v = 1 and $R_2$ is different from H, then u is equal to zero,
w denotes 0 or 1,
A denotes the chain

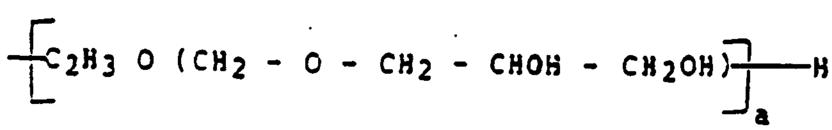

and B denotes the chain

$$-\left[C_2H_3\ O\ (CH_2 - O - CH_2 - CHOH - CH_2OH)\right]_b - H$$

a denotes an integer or decimal from 1.5 to 8,
b denotes zero or an integer or decimal such that a+b=n,
n denotes an integer or decimal from 1.5 to 8.

**7.** Nonionic surface-active products of formula:

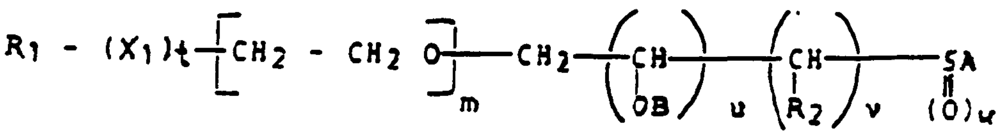 (V)

in which $R_1$ denotes an aliphatic, cycloaliphatic or alkylaryl $C_8$ to $C_{36}$ hydrocarbon radical,
$R_2$ denotes a $C_8$ to $C_{21}$ alkyl or alkoxymethyl radical or a hydrogen atom,
the sum of the carbon atoms in $R_1$ and $R_2$ is not less than 8 and not more than 36,
$X_1$ denotes -O-, -S- or S=O when w=1
m denotes zero or an integer or decimal from 1 to 20,
u denotes zero,
t and v denote zero or the number 1,
w denotes 0 or 1,
A denotes the chain

$$-\left[C_2H_3\ O\ (CH_2 - O - CH_2 - CHCH - CH_2OH)\right]_a - H$$

a denotes an integer or decimal from 1.5 to 8.

**8.** Nonionic surface-active products according to Claim 7, characterised in that $R_1$, $R_2$, t, m, v, w and a have the meanings below:
(i) $R_1 = C_{10}H_{21}$; $R_2$ = H; m=t=w=zero; v=1; a=1.5
(ii) $R_1 = C_{10}H_{21}$; $R_2$ = H; m=t=zero; v=w=1; a=1.5
(iii) $R_1 = C_{17}H_{35}$; m=t=v=w=zero; a=2.

**9.** Composition comprising one or more compounds of formula (V) which are prepared according to Claims 6 to 8, in an appropriate vehicle.

**10.** Cosmetic or pharmaceutical composition characterised in that it comprises one or more compounds of formula (V) according to Claims 6 to 8 in an appropriate vehicle.

**11.** Composition according to Claim 9, which is intended for the textile industry.

**12.** Composition according to Claim 9 or 10, characterised in that it also contains one or more products chosen from nonionic surface-active agents, ionic surface-active agents, natural and synthetic polymers, inorganic, animal or vegetable oils and waxes, polysiloxane derivatives, solvents, propellants, thickeners, preservatives, sunscreen agents, colorants, perfumes, emulsifying agents, steroids, moisturising products, products which are active for the treatment of disorders of the skin or the scalp, and products for hair regrowth.

**13.** Composition according to Claims 9, 10 and 12, characterised in that it is provided in the form of an aqueous solution, dilute alcoholic lotion, oil-in-water or water-in-oil emulsion, microemulsion, micro-dispersions of lipid vesicles containing or not containing active products, gel, cake or sprays.

**Claims for the following Contracting State : ES**

1. Process for preparing nonionic surface-active products from 1,2-isopropylidene-3-epoxyropylglycerol, comprising, in a first stage, reacting a compound of formula (I):

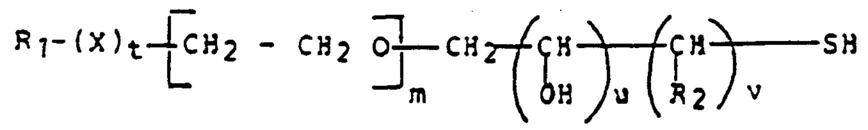

(I)

in which:

$R_1$ denotes an aliphatic, cycloaliphatic or alkylaryl $C_8$ to $C_{36}$ hydrocarbon radical,
$R_2$ denotes a $C_8$ to $C_{21}$ alkyl or alkoxymethyl radical or a hydrogen atom,
X denotes -O- or -S-,
m denotes zero or an integer or decimal from 1 to 20,
t, u and v denote zero or the number 1,
when u = 1, v is necessarily equal to 1 and $R_2$ = H, and
when v = 1 and $R_2$ is different from H, then u is equal to zero, the sum of the carbon atoms in $R_1$ and $R_2$ is not less than 8 and not more than 36,
with 1,2-isopropylidene-3-epoxyropylglycerol of formula:

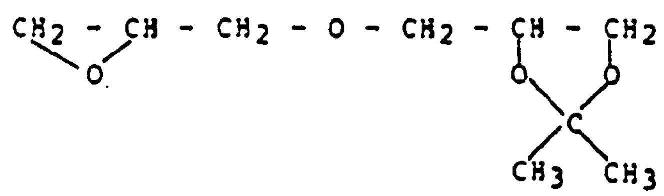

(II)

and, in a second stage, hydrolysing the condensation products, it being possible for the mercaptan functional group to be oxidised to a sulphoxide with hydrogen peroxide, characterised in that more than one mole of epoxide is reacted per mole of mercaptan (n > 1) and in that the molar amount of epoxide of formula (II), equivalent to the molar amount of mercaptan, is added at a temperature of between 40 and 90°C, and in that the remaining amount of epoxide is progressively added over 1 to 3 hours, at a temperature of between 130 and 160°C, after removing the solvent where appropriate.

2. Process according to Claim 1, characterised in that the polyaddition of the epoxide of formula (II) to the mercaptan of formula (I) is carried out in the presence of a basic catalyst such as sodium or potassium methoxide or ethoxide or potassium t-butoxide, at a temperature of between 40 and 160°C.

3. Process according to Claim 1, characterised in that the first addition of epoxide is carried out in the presence of a polar solvent.

4. Process according to Claim 3, characterised in that a $C_1$-$C_4$ alcohol is used as polar solvent.

5. Process according to Claim 1, characterised in that the first addition of epoxide is carried out in a nonpolar solvent such as toluene.

6. Process for preparing nonionic surface-active products according to Claims 1 to 5, characterised in that the compounds of formula (V):

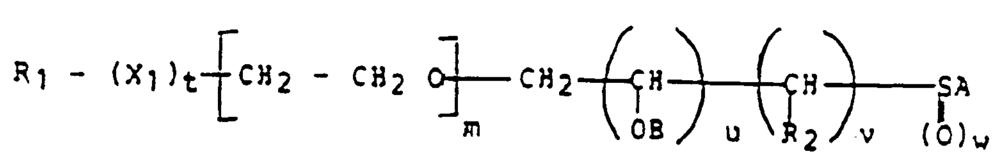

(V)

are prepared in which $R^1$ denotes an aliphatic, cycloaliphatic or alkylaryl $C_8$ to $C_{36}$ hydrocarbon radical,

$R_2$ denotes a $C_8$ to $C_{21}$ alkyl or alkoxymethyl radical or a hydrogen atom,
the sum of the carbon atoms in $R_1$ and $R_2$ is not less than 8 and not more than 36,
$X_1$ denotes -O-, -S- or S=O when w=1,
m denotes zero or an integer or decimal from 1 to 20,
t, u and v denote zero or the number 1,
when u = 1, v is necessarily equal to 1 and $R_2$ = H, and
when v = 1 and $R_2$ is different from H, then u is equal to zero,
w denotes 0 or 1,
A denotes the chain

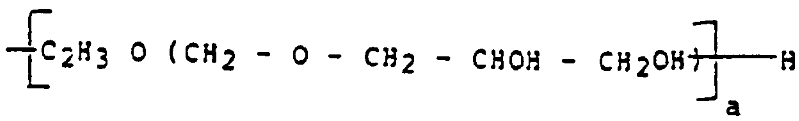

and B denotes the chain

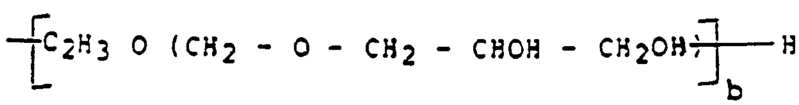

a denotes an integer or decimal from 1.5 to 8,
b denotes zero or an integer or decimal such that a+b=n,
n denotes an integer or decimal from 1.5 to 8.

**7.** Process for preparing nonionic surface-active products of formula (V) according to Claims 1 to 5, characterised in that compounds of formula:

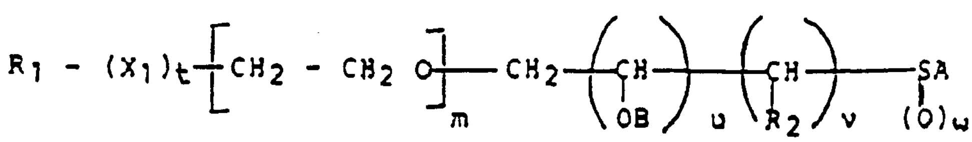 (V)

are prepared in which $R_1$ denotes an aliphatic, cycloaliphatic or alkylaryl $C_8$ to $C_{36}$ hydrocarbon radical,

$R_2$ denotes a $C_8$ to $C_{21}$ alkyl or alkoxymethyl radical or a hydrogen atom,
the sum of the carbon atoms in $R_1$ and $R_2$ is not less than 8 and not more than 36,
$X_1$ denotes -O-, -S- or S=O when w=1
m denotes zero or an integer or decimal from 1 to 20,
u denotes zero,
t and v denote zero or the number 1,
w denotes 0 or 1,
A denotes the chain

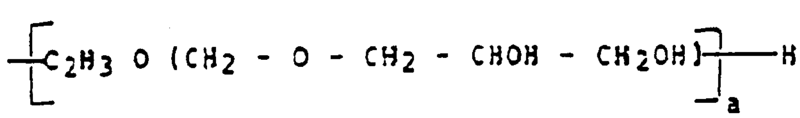

a denotes an integer or decimal from 1.5 to 8.

**8.** Process for preparing nonionic surface-active products of formula (V), according to Claim 7, characterised in that $R_1$, $R_2$, t, m, v, w and a have the meanings below:

(i) $R_1$ = $C_{10}H_{21}$; $R_2$ = H; m=t=w=zero; v=1; a=1.5
(ii) $R_1$ = $C_{10}H_{21}$; $R_2$ = H; m=t=zero; v=w=1; a=1.5

(iii) $R_1 = C_{17}H_{35}$; m=t=v=w=zero; a=2.

**9.** Composition comprising one or more compounds of formula (V) which are prepared according to Claims 6 to 8, in an appropriate vehicle.

**10.** Cosmetic composition characterised in that it comprises one or more compounds of formula (V) in an appropriate vehicle.

**11.** Composition according to Claim 9, which is intended for the textile industry.

**12.** Composition according to Claim 9 or 10, characterised in that it also contains one or more products chosen from nonionic surface-active agents, ionic surface-active agents, natural and synthetic polymers, inorganic, animal or vegetable oils and waxes, polysiloxane derivatives, solvents, propellants, thickeners, preservatives, sunscreen agents, colorants, perfumes, emulsifying agents, steroids, moisturising products, products which are active for the treatment of disorders of the skin or the scalp, and products for hair regrowth.

**13.** Composition according to Claims 10 and 12, characterised in that it is provided in the form of an aqueous solution, dilute alcoholic lotion, oil-in-water or water-in-oil emulsion, microemulsion, microdispersions of lipid vesicles containing or not containing active products, gel, cake or sprays.